# EUROPEAN PATENT APPLICATION

(11) **EP 4 484 542 A1**
(43) Date of publication of application: **01.01.2025**
(21) Application number: 23181657.0
(22) Date of filing: 27.06.2023
(51) Int. Cl.: C12M 1/12, C12M 1/34, C12M 1/36, G01N 27/28, G01N 27/416

(54) **A SYSTEM FOR CALIBRATION OF A SENSOR FOR USE OF THE SENSOR IN A BIOREACTOR AND A METHOD THEREOF**

(71) Applicant: Imec VZW, 3001 Leuven (BE)
(72) Inventor: VAN LANDEGHEM, Nikte, 1050 Brussels (BE); PAEPS, Filip, 3061 Leefdaal (BE)
(74) Representative: AWA Sweden AB

(57) **Abstract**

According to an aspect of the present inventive concept there is provided a system for calibration of a sensor for use of the sensor in a bioreactor, said system comprising: a calibration packaging adapted for carrying a sterile calibration liquid and defining a calibration space within the calibration packaging; a sensor packaging comprising a sensor carrier carrying the sensor and an enclosure enclosing a sensor packaging space in which the sensor carrier is arranged, wherein the sensor packaging and the calibration packaging are configured for allowing moving of the sensor carrier to insert the sensor from the sensor packaging space into the calibration space through a sterile connection between the sensor packaging and the calibration packaging; wherein the sensor is adapted to be subject to a calibration measurement when arranged in the calibration space.

## Description

### Technical field

The present description relates to a system for calibration of a sensor for use of the sensor in a bioreactor and a method for calibrating of a sensor for use of the sensor in a bioreactor.

### Background

Bioreactors can be used for many different applications, where an active biological environment is needed. The bioreactor may be for single use or for multiple use.

To control the process in the bioreactor, sensors are usually used. The sensors need to be calibrated for proper use. Before use, the bioreactor and the sensor must be sterilized. However, sterilization can influence the sensor behavior, such that recalibration is needed. This may be complicated since calibration needs to be performed without breaking the sterility of the sensor. In particular, in some systems, the sensor needs to be sterilized with the bioreactor. This may imply that measurements may not be reliable as the sterilization may have affected the calibration of the sensor. However, if recalibration of the sensor is to be performed, such re-calibration may need to be performed inside the bioreactor, which may limit the calibration performed, e.g., limiting to one-point calibration based on a single solution having known characteristics.

Thus, there is a need in the art for improvements.

### Summary

An objective of the present description is to provide a system for calibration of a sensor for use of the sensor in a bioreactor. A further objective is to provide a system for insertion of a sensor into a bioreactor. Further, an objective is to provide a method for sterile calibration of a sensor for use in a bioreactor.

This and other objectives of the present inventive concept are at least partly met by the invention as defined in the independent claims. Preferred embodiments are set out in the dependent claims.

According to a first aspect, there is provided a system for calibration of a sensor for use of the sensor in a bioreactor, said system comprising:
a calibration packaging adapted for carrying a sterile calibration liquid and defining a calibration space within the calibration packaging;
a sensor packaging comprising a sensor carrier carrying the sensor, an enclosure enclosing a sensor packaging space in which the sensor carrier is arranged, wherein the sensor packaging and the calibration packaging are configured for allowing moving of the sensor carrier to insert the sensor from the sensor packaging space into the calibration space through a sterile connection between the sensor packaging and the calibration packaging;
wherein the sensor is adapted to be subject to a calibration measurement when arranged in the calibration space.

Thanks to the use of the calibration packaging, the sensor may be calibrated before insertion into a bioreactor. The sensor packaging and the calibration packaging provide a sterile connection such that a sterile environment is not breached when the sensor packaging and the calibration packaging are connected. For instance, the pH reading of the sensor may be adjusted. In other words, the calibration curve of the sensor is adjusted. Thus, the sensor may be sterilized before connection to the bioreactor and then calibrated before the insertion into the bioreactor.

By this, the sensor characteristics are less affected than prior art methods. The sensor readings are improved by the system, since it allows for a two-point calibration, where the first calibration is performed in the calibration packaging and the second calibration is performed in the bioreactor.

It should be realized that the system may be used for different bioreactors. Thus, the bioreactor may be a multiple use bioreactor. The bioreactor may be a single use bioreactor.

The calibration packaging is adapted for carrying a sterile calibration liquid. The sterile calibration liquid may be chosen by the user, it may be a pH buffer or cell culture medium. The calibration packaging enables a first point calibration of the sensor to be performed within the calibration space.

The sensor packaging comprises a sensor carrier carrying a sensor. The sensor may be carried at the tip of the sensor carrier or at any side of the sensor carrier. The sensor carrier may have different geometries.

It should be realized that in some embodiments active components of a sensor are arranged such that the sensor in itself forms a sensor carrier. Thus, a separate sensor carrier for carrying the sensor may not necessarily be needed.

Further, the sensor packaging comprises an enclosure which encloses the sensor packaging space in which the sensor carrier is arranged. In other words, the enclosure provides a space in which the sensor carrier may be arranged. The enclosure may be arranged surrounding the sensor carrier along the sensor carrier. The enclosure may permit gases to pass through a material of the enclosure. An advantage of this is that gas-based sterilization methods may be used.

The enclosure may further have a handle at a side of the sensor packaging such that the sensor packaging may be easily handled. The handle may further provide a function of fixating the sensor carrier in the sensor packaging.

The sensor packaging may ensure that a sterile environment is kept within the sensor packaging in the sensor packaging space. The calibration packaging may ensure that a sterile environment is kept within the calibration packaging in the calibration space.

The sensor packaging and the calibration packaging are configured to form a sterile connection between the sensor packaging space and the calibration space. This may be achieved by the sensor packaging being integrated with the calibration packaging forming coherent wall(s) for defining the sensor packaging space and the calibration space. A valve may be used between the sensor packaging space and the calibration space in order to provide selective opening and closing of the connection between the sensor packaging space and the calibration space.

Alternatively, the sensor packaging and the calibration packaging may be separate units, which may be connected to form the sterile connection between the sensor packaging space and the calibration space.

The sensor is adapted to be subject to a calibration measurement when arranged in the calibration space. In this way, the sensor may be calibrated at a time when it is suitable before insertion of the sensor into a bioreactor.

According to one embodiment the calibration packaging may further comprise a first calibration connector end, the first calibration connector end defining an access to the calibration space, the sensor packaging may further comprise a sensor packaging end, the sensor packaging end may be adapted for forming a sterile connection to the first calibration connector end for connecting the sensor packaging to the calibration packaging, the sensor packaging end and the first calibration connector end may be adapted for allowing insertion of the sensor carrier through a connected sensor packaging end and a first calibration connector end into the calibration space.

In other words, the calibration packaging is defining a calibration space within the calibration packaging. The calibration packaging may have a first calibration connector end. The first calibration connector end may define an access to the calibration space. The first calibration connector end may be used for connecting other parts of the system to the calibration packaging.

The sensor packaging may further comprise a sensor packaging end. The sensor packaging end may define an access to the sensor packaging space. The sensor on the sensor carrier may be arranged close to the sensor packaging end, such that when opening the sensor packaging end the sensor may easily be exposed through the sensor packaging end. Further, the sensor packaging end may be closed, such that a sterile environment is kept within the sensor packaging.

In other words, the sensor packaging may comprise a sensor carrier carrying a sensor, a sensor packaging defining a sensor space and a sensor packaging end. The sensor carrier may be inserted through a connected sensor packaging end and a first calibration connector end, such that the sensor carrier carrying the senor may insert the sensor from the sensor packaging to the calibration packaging through the connected sensor packaging end and first calibration packaging end.

According to one embodiment the calibration packaging may further comprise a second calibration connector end separate from the first calibration connector end, wherein the second calibration connector end defines a separate access to the calibration space and is adapted to form an sterile connection between the calibration space and an interior of the bioreactor, wherein the sensor carrier is adapted for allowing insertion of the sensor carrier through the calibration space and the second calibration connector end into the bioreactor.

If the calibration packaging comprises a first calibration connector end, the second calibration connector end are separate from the first calibration connector end. Thus, if the calibration packaging comprises a first and a second calibration connector end, each ends defines a separate access to the calibration space.

In other words, the sensor carrier may insert the sensor into the calibration space through the connected sensor packaging end and the first calibration connector end. While inside the calibration packaging, the sensor may be calibrated before further insertion to the inside of the bioreactor, through the second calibration connector end.

According to one embodiment the calibration packaging may define a straight channel of the calibration space between: the sterile connection between the sensor packaging and the calibration packaging, and the second calibration connector end, and wherein the calibration packaging further defines a side branch connecting to the straight channel of the calibration space, wherein the calibration packaging is adapted to collect the sterile calibration liquid in the side branch.

In other words, the calibration packaging may have different geometries. The geometries may be adapted for letting the sensor meet the calibration liquid, but then to remove the calibration liquid from the sensor. An advantage of this is that the calibration liquid may be removed before the sensor may be inserted further into the bioreactor, such that no or a limited amount of calibration liquid is inserted into the bioreactor.

According to one embodiment the side branch may be adapted to be closed for closing access to the channel of the calibration space through the side branch.

An advantage of this is that the calibration liquid may be collected in the side branch, while sterility is kept since the entire packaging is closed to the outside.

The side branch may be connected to a reservoir. In such a case, the access from the side branch to the reservoir may be closed.

According to one embodiment the system may further comprise an adaptor being hollow defining an adaptor channel within the adaptor, a first adaptor end adapted for being connected to the bioreactor and forming a connection between the adaptor channel and an interior of the bioreactor, and a second adaptor end defining an access to the adaptor channel, wherein the second calibration connector end may be adapted for forming an sterile connection to the second connector end for forming the sterile connection between the calibration space and the interior of the bioreactor via the adaptor.

An advantage of the adaptor is that the sensor packaging and the calibration packaging may be sterilized before connecting it to the bioreactor, via the adaptor. Thus, the sensor packaging including the sensor may be sterilized in a way suitable for the sensor, while the bioreactor is sterilized in any conventional way. The adaptor may be sterilized with the bioreactor. Due to the sterile connections at the first adaptor end and at the second adaptor end, the sterility of the sensor carrier and the sensor can be kept until reaching the inside of the bioreactor.

It should be realized that the adaptor may have different shapes. The adaptor is hollow, such that it defines a channel within the adaptor. The channel may be adapted for insertion of the sensor carrier through the adaptor. The first adaptor end may be configured for being connected to the bioreactor. The connection may form a connection between the channel within the adaptor and the interior of the bioreactor. After sterilization, the connection may be kept sterile, such that any sterile sensor inserted through the adaptor keeps its sterility. The adaptor may be connected to the bioreactor before sterilization and then be sterilized together with the bioreactor.

The adaptor may have a second adaptor end configured for defining an access to the channel. The second adaptor end is adapted for forming a sterile connection to the second calibration connector end and thereby to the calibration space. By the connection of the calibration space to the sensor packaging space, the sensor carrier is further also in connection with the adaptor. Thus, the second adaptor end provides a way of providing a sensor into the bioreactor via the adaptor. In that way, a connection from the sensor packaging, through the calibration packaging to the adaptor may be configured.

Both the second adaptor end and the first adaptor end are configured such that establishing a connection to the adaptor end does not destroy the sterility. Thus, neither of the openings opens the channel of the adaptor to the outside environment.

The adaptor may be configured to provide an interface into the bioreactor facilitating insertion of the sensor into the bioreactor. The adaptor may thus provide an interface allowing a sensor that is separately sterilized to be inserted into the bioreactor. The adaptor may be configured to provide a small-size add-on to an existing bioreactor for providing an improved freedom of insertion of sensors into the bioreactor.

As is already discussed, the sensor carrier may have different geometries. The geometry of the sensor carrier may be adapted to the geometry of the adaptor, such that the hollow part of the adaptor closely encloses the sensor carrier while inserted into the adaptor.

The adaptor may be configured to lock the sensor carrier in a certain rotation before allowing insertion of the sensor carrier into the bioreactor. Thus, the adaptor may have a system for locking the orientation of the sensor carrier. Such system may be a tri clamp system, being arranged between the first adaptor end and the second adaptor end, such that the sensor carrier is inserted through the connected second calibration connector end and second adaptor end, before the orientation of the sensor carrier is fixed. Alternatively, such system may be a barbed connection using a tube connected with tie wraps. Then, the sensor carrier is further inserted through the connected first adaptor end and the bioreactor.

The adaptor may further comprise a closure device. The closure device may be configured to enclose the adaptor, at a position between the first adaptor end and the second adaptor end. The closure device may be used to seal the adaptor to the sensor carrier, such that biological material from the inside of the bioreactor may not leak into the sensor packaging.

The adaptor may be adapted to be screwed to the bioreactor. The connection of the first adaptor end to the bioreactor may be made in several ways. One way is to screw the adaptor on to the bioreactor. The connection may be made using a screw with PG13.5 tread size. This may ensure that the adaptor is adapted to a commonly used interface of the bioreactor.

The sterility of the connection may be further ensured by having an O-ring between the adaptor and the bioreactor.

According to one embodiment the sensor and sensor packaging space may be adapted to be sterilized before connecting the sensor packaging to the calibration packaging.

Thus, the sensor and the sensor packaging may be sterilized in any way suitable for the specific sensor. After sterilization, the calibration may be performed, ensuring proper calibration of the sensor.

The system may be adapted to be used with the bioreactor exhibiting a sterile inside environment. In other words, the bioreactor and optionally the adaptor may be sterilized before the connection to the sterile sensor packaging.

According to one embodiment the sensor may be adapted for measuring physical parameters, chemical parameters or biochemical parameters. Physical parameters may for instance be temperature, conductivity and cell density. Chemical parameters may for instance be pH, ions, dissolved gasses such as oxygen. Biochemical parameters may for instance be protein contents, contaminants, metabolites such as glucose.

Further, more than one sensor may be arranged on the sensor carrier, such that more than one parameter may be measured at the same time.

According to one embodiment the calibration packaging may comprise a first sterile connector at the first calibration connector end and the sensor packaging comprises a second sterile connector at the sensor packaging end.

Thus, the sterile connection between the sensor packaging end and the second adaptor end may be formed by sterile connectors arranged at the respective ends. The sterile connectors may be configured to allow being attached without breaking the sterility and may further allow a connection to be formed through the connected ends in an internal environment for which sterility is maintained. Thus, the sensor packaging may be connected to the adaptor while maintaining a sterile inside environment. The sterile connectors may be adapted for the sensor carrier to be inserted through the connected ends.

According to one embodiment the first calibration packaging end and the sensor packaging end may be adapted for forming the sterile connection by sterile connection welding. This may be an alternative manner for achieving the sterile connection.

According to one embodiment the enclosure may be a collapsible enclosure. Thus, the enclosure may collapse during the insertion of the sensor carrier through a connected sensor packaging end and first calibration connection end. Further, the enclosure may collapse during an insertion of the sensor carrier through a connected second calibration connection end to the bioreactor or to the second adaptor end into the bioreactor, optionally through the channel of the adaptor. This may be suitable for allowing control of the sensor carrier while moving the sensor carrier a substantial distance within the sterile environment to insert the sensor into the bioreactor.

According to another aspect, there is provided a bioreactor sensor system, comprising:
a system according to the first aspect,
a bioreactor defining an interior supporting a biologically active environment;
wherein the bioreactor is adapted to be sterilized for sterilizing the interior before the sensor packaging is connected to the bioreactor;
wherein the sensor and the sensor packaging space are adapted to be sterilized before insertion of the sensor into the bioreactor, and
wherein the sensor is adapted to be subject to a calibration measurement when arranged in the calibration space before insertion of the sensor into the bioreactor.

This aspect may generally present the same or corresponding advantages as the former aspect.

The bioreactor may be a single use bioreactor. The bioreactor may be a multiple use bioreactor.

There may be several advantages with using the present bioreactor sensor system. Any sensor may be used, regardless of the requirement of sterilization method. Thus, the biologically active environment inside the bioreactor may be measured by the sensor in a plurality of ways.

In addition, the sensor may be subject to a calibration measurement before insertion of the sensor into the bioreactor. This implies that effects on the sensor by the sterilization may be at least partially accounted for through the calibration measurement.

According to another aspect, there is provided a method for calibrating of a sensor for use of the sensor in a bioreactor, said method comprising:
connecting a sensor packaging to a calibration packaging;
   wherein the sensor packaging comprises a sensor carrier carrying the sensor, and an enclosure enclosing a sensor packaging space in which the sensor carrier is arranged;
   wherein the calibration packaging is carrying a sterile calibration liquid and defining a calibration space within the calibration packaging;
wherein the sensor and the sensor packaging space are pre-sterilized before connecting the sensor packaging to the calibration packaging or sterilized after connecting the sensor packaging to the calibration packaging;
inserting the sensor carrier through the connected sensor packaging and calibration packaging into the calibration space;
performing a calibration measurement by the sensor using the sterile calibration liquid in the calibration space.

This aspect may generally present the same or corresponding advantages as the former aspect.

The method allows for calibration of a sensor before the sensor is to be used in a bioreactor. The calibration may be made after a sterilization, allowing the sterile sensor to be calibrated outside of the bioreactor before insertion into the bioreactor.

A sterile connection is provided between the sensor packaging space and the calibration space. This may be achieved by a sterile connection being formed between the sensor packaging and the calibration packaging. Alternatively, the sensor packaging and the calibration packaging may be integrated forming a sterile connection, and a valve may be used for selectively closing or opening connection between the sensor packaging space and the calibration space.

The sensor and the sensor packaging are pre-sterilized before the sensor packaging space is connected to the calibration space. By this pre-sterilization, the sensor may drift from the calibration curve and thereby needs to be calibrated again before use. This is possible thanks to the sterile connection between the sensor packaging space and the calibration space.

The sensor carrier is inserted through the sterile connection from the sensor packaging space into the calibration space. With the sensor arranged in the calibration space, the calibration measurement may be performed using the sterile calibration liquid.

According to one embodiment, the calibration packaging may further comprise a first calibration connector end, the first calibration connector end may define an access to the calibration space, the sensor packaging may further comprise a sensor packaging end, the connecting of the sensor packaging may comprise connecting the sensor packaging end to the first calibration connector end for forming a sterile connection between the sensor packaging space and the calibration space.

Thus, the connection of the calibration packaging to the sensor packaging may be made through connecting a first calibration connector end to a sensor packaging end.

According to one embodiment the method may comprise emptying of the sterile calibration liquid from the calibration space by rotating the calibration packaging for allowing the sterile calibration liquid to flow from the calibration space into a side branch.

By this, is it ensured that the calibration liquid is emptied from the calibration space in a controlled way. The side branch may further be closed, such that the calibration liquid is kept inside of the side branch. This limits the risk of the calibration liquid to run back into the calibration space and thereby entering the bioreactor.

According to one embodiment the method may further comprise connecting the calibration packaging to a bioreactor via an adaptor, comprising;
connecting the adaptor to the bioreactor, the adaptor being hollow defining an adaptor channel within the adaptor, a first adaptor end being connected on to the bioreactor and a second adaptor end defining an access to the adaptor channel, wherein the connecting of the adaptor to the bioreactor forms a connection between the adaptor channel and an interior of the bioreactor;
connecting the second calibration connector end to the second adaptor end, for forming a sterile connection between the sensor packaging, through the calibration space and to the interior of the bioreactor via the adaptor;
inserting the sensor carrier through the connected second calibration connector end and the second adaptor end and through the adaptor channel of the adaptor into the bioreactor.

The adaptor may be used for connecting the sensor packaging, through the calibration space, to a bioreactor. This allows for adapting the adaptor to the bioreactor, such that the second calibration connector end may be connected to several different bioreactor having different kind of connectors.

According to one embodiment the method may further comprise re-using the sensor of the sensor packaging for multiple uses of the bioreactor, wherein the method further comprises, after a first use of the bioreactor and the sensor, preparing the bioreactor and the sensor for a second use, said preparing comprising:
removing the calibration packaging from the bioreactor;
sterilizing the bioreactor while the adaptor is connected to the bioreactor;
separately sterilizing the sensor packaging system comprising a sensor, and the calibration packaging;
re-connecting the calibration packaging and the sensor packaging to the adaptor, wherein the re-connecting of the calibration packaging to the adaptor forms a sterile connection between the second calibration connector end and the second adaptor end for re-connecting the sensor packaging space to the adaptor channel of the adaptor;
re-inserting the sensor carrier into the calibration space;
re-calibrating the measurement by the sensor using the sterile calibration liquid in the calibration space
re-inserting the sensor carrier through the re-connected second calibration packaging end and the second adaptor end and through the channel of the adaptor into the bioreactor.

Thanks to the method, the sensor and the sensor packaging may be re-used. Thus, less material may be used. For re-using the sensor and the sensor packaging, the bioreactor and the sensor must, respectively, be prepared before the re-use.

The sensor packaging is removed from the calibration packaging, before closing the sensor packaging end and the second adaptor end.

The calibration packaging is removed from the bioreactor.

The bioreactor is sterilized, while the adaptor is still connected. The bioreactor may be sterilized using autoclave, radiation or chemical sterilization.

Further, the sensor packaging comprising a sensor is separately sterilized by a method adapted for the sensor.

When the bioreactor and the sensor packaging is sterilized, the sensor packaging is re-connected to the calibration packaging. Further, the calibration packaging is connected to the adaptor.

By this, the sensor carrier may be inserted into the calibration space for re-calibrating the measurement by the sensor.

Further, the sensor carrier is re-inserted through the adaptor into the bioreactor.

Optionally, if the sensor packaging and the calibration packaging are connected in a removable way, the sensor packaging may first be removed from the calibration packaging. Optionally, the sensor packaging end may be closed, such that the sensor may be sterilized yet again without contamination from the outside.

Further, after separately sterilizing the sensor packaging system comprising a sensor, and the calibration packaging but before re-connecting the calibration packaging and the sensor packaging to the adaptor, the method may comprise re-connecting the sensor packaging to the calibration packaging, wherein the re-connecting of the sensor packaging to the calibration packaging forms an aseptic sterile connection between the sensor packaging space and the calibration space.

According to one embodiment the sensor packaging may be pre-sterilized using high temperature, radiation or through chemical sterilization.

The sterilization method is adapted to be suitable for the sensor.

According to one embodiment the calibration packaging may be pre-sterilized, optionally wherein the pre-sterilization is made together with the sensor packaging.

By this, the calibration packaging is as sterile as the sensor packaging.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1 is a side view according to an embodiment of the invention.
Fig. 2 is a side view according to an embodiment of the invention.
Fig. 3 is a side view according to an embodiment of the invention.
Fig. 4 is a schematic view according to an embodiment of the invention.
Fig. 5 is a schematic view according to an embodiment of the invention.

### Detailed description

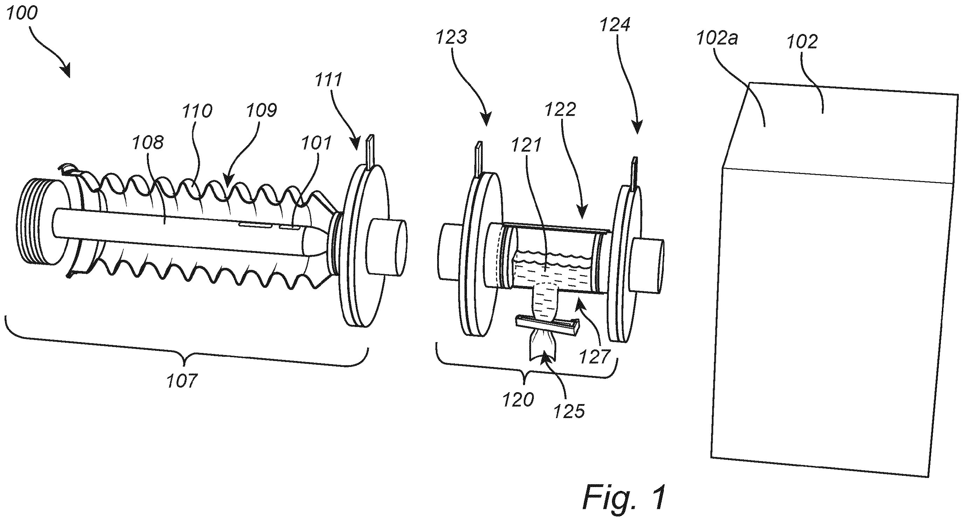

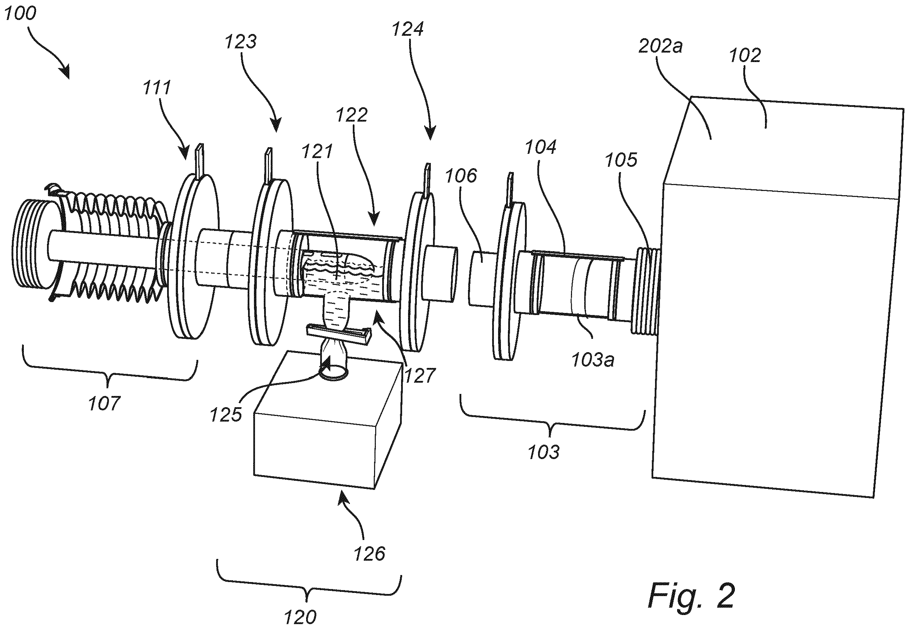

Fig. 1 and Fig. 2 illustrates a system 100 for calibration of a sensor 101 for use of the sensor 101 in a bioreactor 102. The system 100 may be used for different types of bioreactors 102, such as single use bioreactors or multiple use bioreactors.

The system 100 comprises a calibration packaging 120. The calibration packaging is adapted for carrying a sterile calibration liquid 121 and defines a calibration space 122 within the calibration packaging 120.

In Fig. 1 and Fig 2, the calibration packaging 120 has a first calibration connector end 123. The first calibration connector end 123 defines an access to the calibration space 122. However, it should be understood that the function of the first calibration connector end may be acquired in other ways discussed below.

The calibration packaging 120 may further comprise a second calibration connector end 124 separate from the first calibration connector end 123. The relation between the first calibration connection end 123 and the second calibration connector end 124 may be different. However, as is disclosed in Fig. 1 and Fig. 2, the calibration packaging 120 may define a straight channel 127 of the calibration space 122 between the first calibration connector end 123 and the second calibration connector end 124.

The second calibration connector end 124 defines a separate access to the calibration space 122 and is adapted to form a sterile connection between the calibration space 122 and an interior of the bioreactor 202a.

As is disclosed in Fig. 1 and Fig. 2, the calibration packaging 120 may further define a side branch 125 connecting to the straight channel 127 of the calibration space 122. The calibration packaging 120 is adapted to collect the sterile calibration liquid 121 in the side branch 125. The side branch 125 may be adapted to be closed for closing access to the straight channel 127 of the calibration space 122 through the side branch 125. The side branch 125 may be connected to a reservoir 126. In such a case, the access from the side branch 125 to the reservoir 126 may be closed.

The system 100 further comprises a sensor packaging 107 comprising a sensor carrier 108, carrying the sensor 101, an enclosure 109 enclosing a sensor packaging space 110 in which the sensor carrier 108 is arranged. The sensor packaging 107 may further comprise a sensor packaging end 111.

The sensor packaging end 111 is adapted for forming a sterile connection to the first calibration end 123 for connecting the sensor packaging space 110 to the calibration space 122.

However, the connection between the sensor packaging 107 and the calibration packaging 120 may be acquired in other ways.

The sensor packaging 107 and the calibration packaging 120 are configured to form a sterile connection between the sensor packaging space 110 and the calibration space 122. This may be achieved by the sensor packaging 107 being integrated with the calibration packaging 120 forming coherent wall(s) for defining the sensor packaging space 110 and the calibration space 122. A valve may be used between the sensor packaging space 110 and the calibration space 122 in order to provide selective opening and closing of the connection between the sensor packaging space 110 and the calibration space 122.

Alternatively, the sensor packaging 107 and the calibration packaging 120 may be separate units, which may be connected to form the sterile connection between the sensor packaging space 110 and the calibration space 122.

The calibration packaging 120 may comprise a first sterile connector at the first calibration connector end 123 and the sensor packaging 107 comprises a second sterile connector at the sensor packaging end 111.

The first calibration packaging end 123 and the sensor packaging end 111 may be adapted for forming the sterile connection by sterile connection welding.

The sensor carrier 108 is adapted for allowing insertion of the sensor carrier 108 into the calibration space 122.

The sensor 101 is adapted to be subject to a calibration measurement when arranged in the calibration space 122.

The sensor 101 is adapted for measuring physical parameters, chemical parameters or biochemical parameters. Physical parameters may be temperature, conductivity and cell density. Chemical parameters may be pH, ions, dissolved gasses such as oxygen. Biochemical parameters may be protein contents, contaminants, metabolites such as glucose.

More than one sensor 101 may be arranged on the sensor carrier 108, such that more than one parameter may be measured at the same time.

The sensor 101 may be carried at the tip of the sensor carrier 108 or at any side of the sensor carrier. The sensor carrier 108 may have different geometries. The geometry of the sensor carrier 108 may be adapted to the geometry of the adaptor 103, such that the hollow part of the adaptor 103 closely encloses the sensor carrier 108 while inserted into the adaptor 103.

The sensor 101 and the sensor packaging space 110 are adapted to be sterilized before connecting the sensor packaging 107 to the calibration packaging 120.

As is depicted in Fig. 2, the system 100 may further comprise an adaptor 103. The adaptor 103 is hollow, such that it defines an adaptor channel 104 within the adaptor 103. It should be realized that the adaptor 103 may have different shapes. The adaptor channel 104 of the adaptor 103 is adapted for insertion of a sensor carrier 108 through the adaptor 103 into the bioreactor 102.

The adaptor 103 further comprises a first adaptor end 105 configured for being connected to the bioreactor 102. The connection may be made by screwing the first adaptor end 105 to the bioreactor 102. The connection of the first adaptor end 105 to the bioreactor 102 forms a connection between the adaptor channel 104 and an interior of the bioreactor 102.

The adaptor 103 may be sealed to the bioreactor 102 by the use of an O-ring. The O-ring may be arranged in connection with the first adaptor end 105. The O-ring may for instance be arranged such that it is arranged between the first adaptor end 105 and the bioreactor 102, when the first adaptor end 105 is attached to the bioreactor 102.

The adaptor 103 further comprises a second adaptor end 106. The second adaptor end 106 defines an access to the adaptor channel 104.

In other words, the first adaptor end 105 and the second adaptor end 106 may be arranged on opposite sides of the adaptor 103 and provides access from the second adaptor end 106 through the adaptor channel 104 into the bioreactor 102.

The second calibration connector end 124 is adapted for forming a sterile connection to the second adaptor end 106 for forming the sterile connection between the calibration space 122 and the interior 202a of the bioreactor 102 via the adaptor 103.

The adaptor 103 may comprise a sterile connector at the second adaptor end 106. The calibration packaging 120 may comprise a sterile connector at the second calibration connector end 124.

The sterile connector at the second adaptor end 106 and at the calibration connector end 124 may be a type of sterile connector known in the art, such as a CPCO AsetiQuik^{®} Sterile Connector provided by CPC (Colder Products Company). In other words, it may be a sterile connector comprising a membrane preventing the sterile environment to be broken. When the second adaptor end 106 and the calibration connector end 124 are connected to each other, with sterile connectors adapted for forming a close contact to each other, the membrane may be removed such that the connecting forms an opening through the connected ends.

The second adaptor end 106 and the second calibration connector end 124 may be adapted for forming the sterile connection by sterile connection welding.

The connection of the second adaptor end 106 to the second calibration connector end 124 provides a sterile connection from the calibration packaging 120, through the adaptor 103 and into the bioreactor 102.

Fig. 2 discloses how the sensor packaging end 111 and the first calibration connector end 123 are adapted for allowing insertion of the sensor carrier 108 through a connected sensor packaging end 111 and the first calibration connector end 123 into the calibration packaging 120.

The sensor 101 is calibrated by the sterile calibration liquid 121 inside of the calibration packaging 120.

Further, the sensor carrier 108 may be inserted into the bioreactor 102 through a connected adaptor 103.

The adaptor 103 may be configured to lock the sensor carrier 108 in a certain rotation before allowing insertion of the sensor carrier 108 into the bioreactor 102. Thus, the adaptor 103 may have a system for locking the orientation of the sensor carrier 108. Such system may be a tri clamp system, being arranged between the first adaptor end 105 and the second adaptor end 106, such that the sensor carrier 108 is inserted through the connected second calibration connector end 124 and second adaptor end 106, before the orientation of the sensor carrier 108 is fixed. Then, the sensor carrier 108 is further inserted through the connected first adaptor end 105 and the bioreactor 102.

The adaptor 103 may further comprise a closure device 103a. The closure device 103a may be configured to enclose the adaptor, at a position between the first adaptor end 105 and the second adaptor end 106. The closure device 103a may be used to seal the adaptor 103 to the sensor carrier 108, such that biological material from the inside of the bioreactor 102 may not leak into the sensor packaging 107.

At insertion, the enclosure 109 may be adapted for collapsing during insertion into the bioreactor 102.

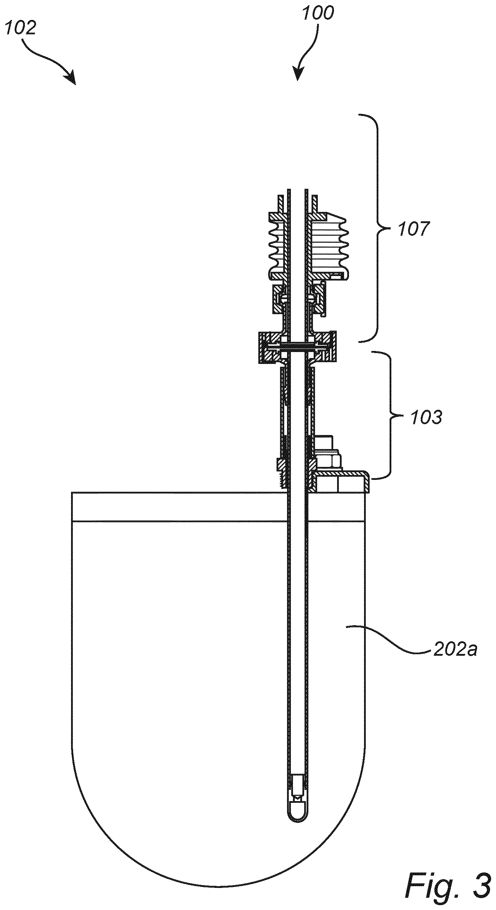

Fig. 3 discloses a bioreactor sensor system 200. The bioreactor system 200 comprises a system 100 according to above.

Further, the bioreactor system 200 comprises a bioreactor 102 defining an interior 202a supporting a biologically active environment. The bioreactor 102 may be adapted for multiple use. The bioreactor 102 may be adapted for single use. The bioreactor 102 may be adapted to be sterilized for sterilizing the interior 202a while the adaptor 103 may be connected to the bioreactor and before the calibration packaging 120 is connected to the adaptor 103.

The sensor 101 and the sensor packaging space 110 are adapted to be sterilized before insertion and before connecting the sensor packaging 107 to the calibration packaging 120. The sensor 101 is calibrated for a first time in the calibration liquid. The calibration space 122 may be connected to the adaptor 103 and the sensor is inserted into the bioreactor 102.

The system provides with an opportunity to use a two-point calibration for the sensor 101. The sensor may first be calibrated in the calibration packaging 120, providing a first point calibration. The calibration packaging 120 may then be connected to a bioreactor 102. Through the connection, the sensor carrier 108 may carry the sensor 101 into the bioreactor 102. The sensor may then be calibrated in the bioreactor 102 using bioreactor culture medium in the bioreactor 102. The calibration measurement in the bioreactor may provide a second point calibration. Having two calibration points may provide a highly improved accuracy of calibration compared to a single point calibration.

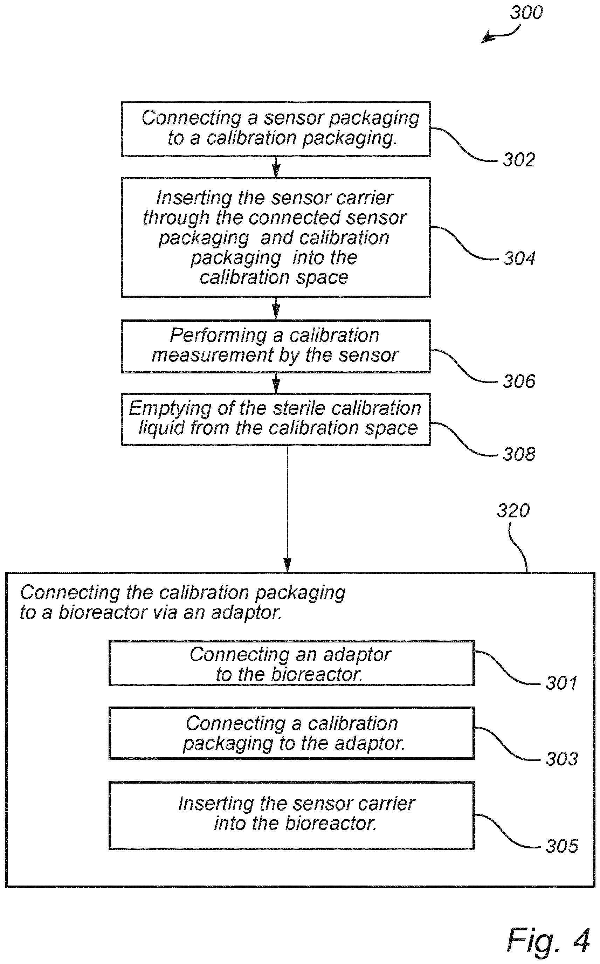

Fig. 4 illustrates a schematic view of a method 300 for calibrating of a sensor 101 for use of the sensor 101 in a bioreactor 102.

The method 300 comprises connecting 302 a sensor packaging 107 to a calibration packaging 120.

As is disclosed above, the sensor packaging 107 comprises a sensor carrier 108 carrying the sensor 101 and an enclosure 109 enclosing a sensor packaging space 110 in which the sensor carrier 108 is arranged. Further, the calibration packaging 120 is carrying a sterile calibration liquid 121 and defining a calibration space 122 within the calibration packaging 120.

Optionally, the sensor packaging 107 may comprise a sensor packaging end 111. Further, optionally, the calibration packaging 120 may have a first calibration connector end 123 defining an access to the calibration space 122.

The connecting 301 of the sensor packaging 107 may comprise connecting the sensor packaging end 111 to the first calibration connector end 123 for forming a sterile connection between the sensor packaging space 107 and the calibration space 122.

However, as is discussed already, the connecting 301 may be made in various ways. The sensor packaging 107 may be integrated with the calibration packaging 120 so as to form coherent wall(s) for defining the sensor packaging space 110 and the calibration space 122. A valve may be used between the sensor packaging space 110 and the calibration space 122 in order to provide selective opening and closing of the connection between the sensor packaging space 110 and the calibration space 122.

Alternatively, the sensor packaging 107 and the calibration packaging 120 may be separate units, which may be connected to form the sterile connection between the sensor packaging space 110 and the calibration space 122.

The connection 302 of the sensor packaging 107 to the calibration packaging 120 may comprise connecting a second sterile connector at the sensor packaging end 111 to a first sterile connector at the first calibration connector end 123. For instance, the sterile connectors may each comprise a membrane, which may be removed after connection of the first calibration connector end 123 to the sensor packaging end 111. This may allow for the sterility of the sensor packaging 107 and the calibration liquid 121 to be kept before the connection of the two parts to each other. The removal of the membrane opens up between the connected ends.

The connection 302 of the sensor packaging 107 to the calibration packaging 120 may comprise connecting the sensor packaging end 111 to the first calibration connector end 123 through sterile welding of the sensor packaging end 111 and the first calibration connector end 123.

The sensor 101 and the sensor packaging space 107 are pre-sterilized before connecting the sensor packaging 107 to the calibration packaging 120. Alternatively, the sensor 101 and the sensor packaging 107 are connected to the calibration packaging 120 before pre-sterilization. The sensor packaging 107 and the calibration packaging 120 may be pre-sterilized using high temperature, radiation or through chemical sterilization. Further, the calibration packaging 120 may be pre-sterilized. This may optionally be made together with the sensor packaging 107. Such methods may be suitable for the sensor 101, but may not be possible to use as a sterilization method for the entire bioreactor 102.

The method 300 further comprises inserting 304 the sensor carrier 108 through the connected sensor packaging 107 and the calibration packaging 120 into the calibration space 122.

Thanks to the sterility of the calibration liquid 121, the sensor is kept sterile while inserted into the calibration space 122. During insertion, the enclosure 109 may collapse to allow insertion of the sensor carrier 108 into the calibration space 122.

The method further comprises performing 306 a calibration measurement by the sensor 101 using the sterile calibration liquid 121 in the calibration space 122. By this, a first point calibration is performed.

The method 300 may further comprise emptying 308 of the sterile calibration liquid 121 from the calibration space 122, for instance by rotating the calibration packaging for allowing the sterile calibration liquid 121 to flow from the calibration space 122 into a side branch 125. It should be understood that this is a closed system, thus, even the emptying 308 of the sterile calibration liquid 121 will keep the sterility of the sensor 101. Thanks to the emptying of the calibration liquid 121 from the calibration space 122, it may be ensured that calibration liquid 121 does not enter the bioreactor when the calibration packaging is further connected to the bioreactor. In addition, a connection between the side branch 125 and the calibration space 122 may be closed after emptying of the calibration liquid 121 from the calibration space 122, e.g. by clamping the side branch 125.

The method 300 may further comprise connecting 320 the calibration packaging 120 to a bioreactor 102 via an adaptor 103. This is made by connecting 301 an adaptor 103 to the bioreactor 102. As is disclosed above, the adaptor 103 is hollow defining an adaptor channel 104 within the adaptor 103. The adaptor 103 further comprises a first adaptor end 105 being connected to the bioreactor 102 and a second adaptor end 106 defining an access to the adaptor channel 104.

The connecting of the adaptor 103 to the bioreactor 102 forms a connection between the adaptor channel 104 and an interior 202a of the bioreactor.

Alternatively, the calibration packaging 120 may be connected to the bioreactor 102 without the use of the adaptor 103. For instance, the calibration packaging end 124 may be adapted such that it can be connected to the bioreactor 102.

The method 300 further comprises connecting 303 the calibration packaging 120 to the adaptor 103. By the use of the adaptor 103, the calibration packaging 120 may be sterilized before the connection 303 to the adaptor 103. Further, the sensor 101 may be calibrated in the calibration packaging 120 before or after the connection 303 to the adaptor 103.

The bioreactor 102 and the adaptor 103 may be sterilize before connection of the calibration packaging 120 to the adaptor 103.

The connecting 303 of the calibration packaging 120 to the adaptor 103 forms a sterile connection between the calibration packaging end 124 and the second adaptor end 106 for connecting the calibration space 122 to the adaptor channel 104 of the adaptor 103.

Further, the connecting 303 of the calibration packaging 120 to the adaptor 103 forms a sterile connection between the sensor packaging 107 being connected to the calibration packaging 120.

The connection 303 of the calibration packaging 120 to the adaptor 103 may comprise connecting a second sterile connector at the calibration packaging end 124 to a first sterile connector at the second adaptor end 106. For instance, the sterile connectors may each comprise a membrane, which may be removed after connection of the second adaptor end 106 to the calibration connector end 124. This may allow for the sterility of the calibration packaging 120 and the adaptor 103 to be kept before the connection of the two parts to each other. The removal of the membrane opens up between the connected ends.

The connection 303 of the calibration packaging 120 to the adaptor 103 may comprise connecting the calibration connector end 124 to the second adaptor end 106 through sterile welding of the calibration connector end 124 and the second adaptor end 106.

The bioreactor exhibits a sterile inside 202a environment. The sterilization of the inside 202a of the bioreactor may be made by any sterilization method, for instance a different sterilization method compared to the sterilization of the sensor 101 and the sensor packaging space 110 and the calibration packaging 120.

The method 300 further comprises inserting 305 the sensor carrier 108 through the connected second calibration connector end 124 and second adaptor end 106 and through the adaptor channel 104 of the adaptor 103 into the bioreactor 102.

During insertion 305, the enclosure 109 may collapse for facilitating moving of the sensor carrier 108 within the sensor packaging space 110, the calibration space 122 and into the bioreactor 102.

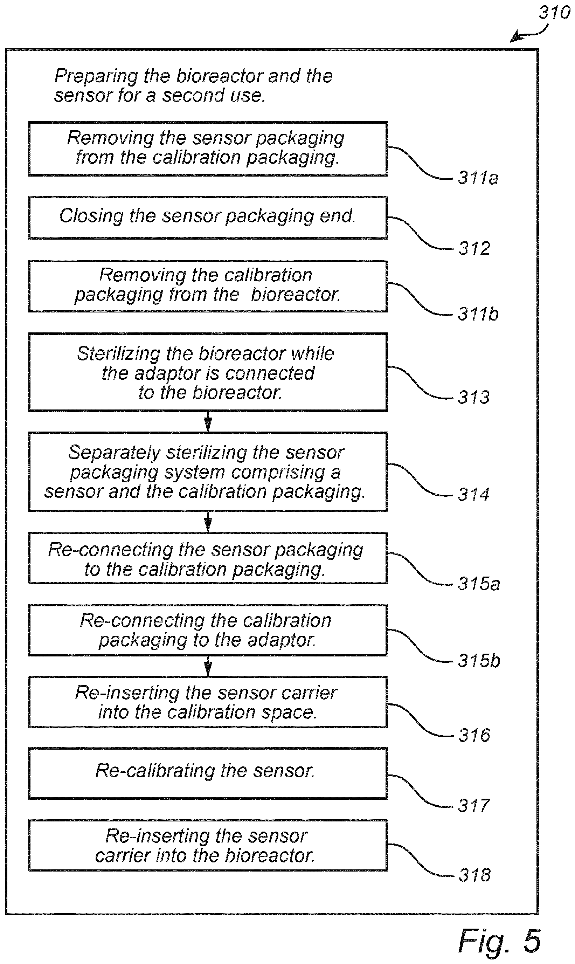

Fig. 5 illustrates that the method 300 may further comprise re-using the sensor 101 of the sensor packaging 107 for multiple uses of the bioreactor 102.

For this, the method 300 further comprises, after a first use of the bioreactor 102 and the sensor 101, preparing 310 the bioreactor 102 and the sensor 101 for a second use.

The preparing 310 comprises removing 311b the calibration packaging from the bioreactor.

Further, the preparing 310 comprises sterilizing 313 the bioreactor 102 while the adaptor 103 is connected to the bioreactor 102 and separately 314 sterilizing the sensor packaging system 107 comprising a sensor 101 and the calibration packaging 120. The preparing 310 may comprise removing the sensor 101 from the sensor carrier 108. If so, a new sensor 101 may be added to the sensor carrier 108 after the sterilization 314.

Further, it should be understood that the same sensor 101 may be used for different bioreactors 102. This is especially relevant for single use bioreactors 102.

Further, the preparing 310 comprises re-connecting 315b the calibration packaging 120 to the adaptor 103. The re-connecting 315b of the calibration packaging 120 to the adaptor 103 forms a sterile connection between the second calibration connector end 124 and the second adaptor end 106 for re-connecting the calibration space 122 to the adaptor channel 104 of the adaptor 103.

Further, the preparing 310 comprises re-inserting 316 the sensor carrier 108 through the re-connected sensor packaging end 111 and the first calibration connector end 123 into the calibration space 122. This is followed by re-calibrating 317 the measurement by the sensor 101 using the sterile calibration liquid 121 in the calibration space 122.

Further, the preparing 310 comprises re-inserting 318 the sensor carrier 108 through the re-connected second calibration connector end 124 and second adaptor end 106 and through the adaptor channel 104 of the adaptor 103 into the bioreactor 102.

The preparing 310 may optionally comprise, before the removing 311b, removing 311a the sensor packaging 107 from the calibration packaging 120. The removal 310 may be made by disconnecting the sensor packaging end 111 from the first calibration connector end 123.

Further, the preparing 310 may optionally comprise closing 312 the sensor packaging end 111 and the second adaptor end 106.

Further, the preparing may optionally further comprise, before re-connecting 315b, re-connecting 315a the sensor packaging 107 to the calibration packaging 120. The re-connecting 315a of the sensor packaging 107 to the calibration packaging 120 forms a sterile connection between the sensor packaging space 110 and the calibration space 122.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A system (100) for calibration of a sensor (101) for use of the sensor (101) in a bioreactor (102), said system (100) comprising:
a calibration packaging (120) adapted for carrying a sterile calibration liquid (121) and defining a calibration space (122) within the calibration packaging (120);
a sensor packaging (107) comprising a sensor carrier (108) carrying the sensor (101), an enclosure (109) enclosing a sensor packaging space (110) in which the sensor carrier (108) is arranged, wherein the sensor packaging (107) and the calibration packaging (120) are configured for allowing moving of the sensor carrier (108) to insert the sensor (101) from the sensor packaging space (110) into the calibration space (122) through a sterile connection between the sensor packaging (107) and the calibration packaging (120);
wherein the sensor (101) is adapted to be subject to a calibration measurement when arranged in the calibration space (122).

2. The system (100) according to claim 1, wherein the calibration packaging (120) further comprises a first calibration connector end (123), the first calibration connector end (123) defining an access to the calibration space (122), wherein the sensor packaging (107) further comprises a sensor packaging end (111), wherein the sensor packaging end (111) is adapted for forming a sterile connection to the first calibration connector end (123) for connecting the sensor packaging (107) to the calibration packaging (120), wherein the sensor packaging end (111) and the first calibration connector end (123) are adapted for allowing insertion of the sensor carrier (108) through a connected sensor packaging end (111) and a first calibration connector end (123) into the calibration space (122).

3. The system (100) according to any one of claim 1 or 2, wherein the calibration packaging (120) further comprises a second calibration connector end (124), wherein the second calibration connector end (124) defines an access to the calibration space (122) and is adapted to form an sterile connection between the calibration space (122) and an interior of the bioreactor (202a), wherein the sensor carrier (108) is adapted for allowing insertion of the sensor carrier (108) through the calibration space (122) and the second calibration connector end (124) into the bioreactor (102).

4. The system (100) according to claim 3, wherein the calibration packaging (120) defines a straight channel (127) of the calibration space (122) between:
the sterile connection between the sensor packaging (107) and the calibration packaging (120), and
the second calibration connector end (124), and
wherein the calibration packaging (120) further defines a side branch (125) connecting to the straight channel (127) of the calibration space (122),
wherein the calibration packaging (120) is adapted to collect the sterile calibration liquid (121) in the side branch (125).

5. The system (100) according to any one of claims 3 or 4, wherein the system (100) further comprises an adaptor (103) being hollow defining an adaptor channel (104) within the adaptor (103), a first adaptor end (105) adapted for being connected to the bioreactor (102) and forming a connection between the adaptor channel (104) and an interior (202a) of the bioreactor (102), and a second adaptor end (106) defining an access to the adaptor channel (104), wherein the second calibration connector end (124) is adapted for forming an sterile connection to the second connector end (106) for forming the sterile connection between the calibration space (122) and the interior (202a) of the bioreactor (102) via the adaptor (103).

6. The system (100) according to any one of the preceding claims, wherein the sensor (101) and sensor packaging space (110) are adapted to be sterilized before connecting the sensor packaging (107) to the calibration packaging (120).

7. The system (100) according to any one of the preceding claims, wherein the sensor (101) is adapted for measuring physical parameters, chemical parameters or biochemical parameters.

8. The system (100) according to claim 2, wherein the calibration packaging (120) comprises a first sterile connector at the first calibration connector end (123), and the sensor packaging (107) comprises a second sterile connector at the sensor packaging end (111).

9. The system (100) according to any one of claims 2, wherein the first calibration packaging end (123) and the sensor packaging end (111) are adapted for forming the sterile connection by sterile connection welding.

10. A method (300) for calibrating of a sensor (101) for use of the sensor (101) in a bioreactor (102), said method comprising:
connecting (302) a sensor packaging (107) to a calibration packaging (120);
wherein the sensor packaging (107) comprises a sensor carrier (108) carrying the sensor (101) and an enclosure (109) enclosing a sensor packaging space (110) in which the sensor carrier (108) is arranged;
wherein the calibration packaging (120) is carrying a sterile calibration liquid (121) and defining a calibration space (122) within the calibration packaging (120);
wherein the sensor (101) and the sensor packaging space (107) are pre-sterilized before connecting the sensor packaging (107) to the calibration packaging (120) or sterilized after connecting the sensor packaging (107) to the calibration packaging (120);
inserting (304) the sensor carrier (108) through the connected sensor packaging (107) and calibration packaging (120) into the calibration space (122);
performing (306) a calibration measurement by the sensor (101) using the sterile calibration liquid (121) in the calibration space (122).

11. The method (300) according to claim 10, wherein the calibration packaging (120) further comprises a first calibration connector end (123), the first calibration connector end (123) defining an access to the calibration space (122), wherein the sensor packaging (107) further comprises a sensor packaging end (111), wherein the connecting (301) of the sensor packaging (107) comprises connecting the sensor packaging end (111) to the first calibration connector end (123) for forming a sterile connection between the sensor packaging space (107) and the calibration space (122).

12. The method (300) according to any one of claim 10 or 11, further comprising emptying (308) of the sterile calibration liquid (121) from the calibration space (122) by rotating the calibration packaging for allowing the sterile calibration liquid (121) to flow from the calibration space (122) into a side branch (125).

13. The method (300) according to any one of claims 10-12, further comprising connecting (320) the calibration packaging (120) to a bioreactor (102) via an adaptor (103), comprising;
connecting (301) the adaptor (103) to the bioreactor (102), the adaptor (103) being hollow defining an adaptor channel (104) within the adaptor (103), a first adaptor end being (105) connected to the bioreactor (102) and a second adaptor end (106) defining an access to the adaptor channel (104), wherein the connecting of the adaptor (103) to the bioreactor (102) forms a connection between the adaptor channel (104) and an interior of the bioreactor (202a);
connecting (303) the second calibration connector end (124) to the second adaptor end (106), for forming a sterile connection between the sensor packaging (107), through the calibration space (122) and to the interior (202a) of the bioreactor (102) via the adaptor (103);
inserting (305) the sensor carrier (108) through the connected second calibration connector end (124) and the second adaptor end (106) and through the adaptor channel (104) of the adaptor (103) into the bioreactor (102).

14. The method (300) according to claim 13, further comprising re-using the sensor (101) of the sensor packaging (107) for multiple uses of the bioreactor (102), wherein the method (300) further comprises, after a first use of the bioreactor (102) and the sensor (101), preparing (310) the bioreactor (102) and the sensor (101) for a second use, said preparing (310) comprising:
removing (311b) the calibration packaging (120) from the bioreactor;
sterilizing (313) the bioreactor (102) while the adaptor (103) is connected to the bioreactor (102);
separately (314) sterilizing the sensor packaging system (107) comprising a sensor (101), and the calibration packaging (120);
re-connecting (315b) the calibration packaging (120) and the sensor packaging (107) to the adaptor (103), wherein the re-connecting (315b) of the calibration packaging (120) to the adaptor (103) forms a sterile connection between the second calibration connector end (124) and the second adaptor end (106) for re-connecting the calibration space (122) to the adaptor channel (104) of the adaptor (103);
re-inserting (316) the sensor carrier (108) into the calibration space (122);
re-calibrating (317) the measurement by the sensor (101) using the sterile calibration liquid (121) in the calibration space (122);
re-inserting (318) the sensor carrier (108) through the re-connected second calibration connector end (124) and second adaptor end (106) and through the adaptor channel (104) of the adaptor (103) into the bioreactor (102).

15. The method (300) according to any one of claims 10-14, wherein the sensor packaging (107) is pre-sterilized using high temperature, radiation or through chemical sterilization.
